# EUROPEAN PATENT SPECIFICATION

(11) **EP 1 157 306 B1**
(45) Date of publication and mention of the grant of the patent: **01.06.2005**
(21) Application number: 00901162.8
(22) Date of filing: 07.01.2000
(51) Int. Cl.: G03B 42/02

(54) **A METHOD AND AN APPARATUS FOR FEEDING IMAGE PLATES USED FOR INTRAORAL DENTAL X-RAY PHOTOGRAPHY INTO A READING DEVICE**
VORRICHTUNG UND VERFAHREN ZUR EINGABE VON BILDPLATTEN FÜR INTRAORALE RÖNTGENOGRAPHIE IN EIN LESEGERÄT
PROCEDE ET APPAREIL D'ACHEMINEMENT DANS UN DISPOSITIF DE LECTURE DE PLAQUES IMAGES UTILISEES DANS UN RADIOGRAMME BUCCAL

(30) Priority: 08.01.1999 FI 990025
(43) Date of publication of application: 28.11.2001
(73) Proprietor: GE Healthcare Finland Oy, 00510 Helsinki (FI)
(72) Inventor: KOVANEN, Ilkka, FIN-00720 Helsinki (FI); TURKUMÄKI, Terho, FIN-04130 Sipoo (FI)
(74) Representative: Saijonmaa, Olli-Pekka
(86) International application number: PCT/FI2000/000010
(87) International publication number: WO 2000/041035

(56) References cited:
- EP-A1- 0 056 638
- EP-A2- 0 219 836
- EP-A2- 0 919 857
- FI-B- 90 471
- FI-B- 92 633
- US-A- 5 148 028
- US-A- 5 340 995
- US-A- 5 635 728
- US-A- 5 818 065

## Description

The invention relates to a method for feeding image plates used in intraoral dental X-ray photography into a reading device for the images taken on the plates to be read. The invention also relates to an apparatus for carrying out the method in question.

In intraoral dental X-ray photography, the image plate inserted into the patient's mouth is exposed to X-radiation in order to excite an excitable layer on the plate, such as a phosphorus layer. After the photographing step, the plate surface is read with a scanning laser beam to produce an image. After the reading step, the image information is removed from the plate by means of a clearing light to allow the plates to be reused.

FI layout print 90471 describes the reading and clearing of an image during a reciprocating movement generated by a pulling device, which attracts the plate magnetically. The publication states that the pulling device retracts the plate from a cassette, in which the plate has been located during the photographing step. FI layout print 92633 further describes a protective bag of plastic film permeable to X-rays, in which the image plate is located during the photographing step. After the photographing step, the plates are transferred one by one to a reading device, the transfer taking place according to the publication so that the magnet included in the pulling device attracts the end of the plate located in the bag, and after this the bag is withdrawn from around the plate.

US patent specification 5,635,728 discloses a solution for reading image plates in connection with dental intraoral X-ray photography, in which the plates are manually fixed one by one in vertical and horizontal rows on the outer surface of a cylindrical, rotatable cassette. The cassette is placed in a reading device, where a scanner reads the plates one horizontal row at the time while the cassette is rotating, or where plates are read one vertical row at the time by shifting the scanner vertically with the cassette immobilised during the reading of each row. This technique has the inconvenience of awkward attachment of the image plates to and detachment from the cassette after the reading, and also of requiring flexible plates. To attach and detach the plates, the cassette has to be detached from the reading apparatus, thus interrupting the reading, unless two or more alternating cassettes are available. In addition, to gain effective benefit by the method, all the plate sites of the cassette should be occupied. If there are unoccupied sites in the plate rows, as there will be if the number of plates to be read is smaller than the number of plate sites, part of the reading capacity will be lost due to idle running of the apparatus, entailing unnecessary costs and waste of time.

Examples of conventional methods and apparatuses for feeding image plates are disclosed in: EP-A-0 919 857, US-A-25 148 028, EP-A-0 219 836, EP-A-0 056 638, US-A-5 818 065, US-A-5 340 995.

The object of the invention is to provide a solution for bringing image plates used in dental intraoral X-ray photography to the image reading step in a manner that overcomes the inconveniences described above. The invention provides a method as defined in claim 1 which can be applied to reading techniques, where a slide acting as a conveyor transfers image plates one by one to be read by a scanner.

It is easy to place image plates to be read in the housing, extending a pack of plates which may already be there, and it can be done freely, independently of the actual reading process. Plates can be added not only one by one but also as bundles of several plates. When the plates are in the housing, they are submitted to further handling, i.e. transferred with a conveyor e.g. within reach of a separate slide in the reading device, and subsequently automatically transferred by the slide to be read by the scanner. Hence the system does not require monitoring, and, unlike the techniques of US patent specification 5,635,728, it only handles and reads plates that have actually been brought into the apparatus, without idle running. The invention does not either necessarily require flexible plates.

It is advantageous to arrange the image plates as a pack in the housing, where an active force pulls or presses the plates towards a conveyor tangential or adjacent to the housing, the conveyor gripping the plate which is closest each time in order to move it forward on the feed path. The image plates in the housing can be pulled or pushed towards the conveyor within reach of the conveyor gripping means in several ways. One advantageous solution is to tilt the housing bottom towards the conveyor so that the plates in the pack are pulled towards the conveyor by force of gravity. Besides or instead of this, the plate pack can be pushed towards the conveyor by means of a pushing device behind the pack. The pushing device may advantageously consist e.g. of a roll rolling freely on the inclined housing bottom. Optionally, the pushing device can be connected to a power source, and then no inclined housing bottom will be needed. One further solution, which can be used separately or as a complement to the solutions mentioned above, is to provide the image plates with a magnetic metal part which is attracted to the conveyor with the aid of a magnet.

A conveyor on which image plates are transported to the path of the reader slide is advantageously formed by a moving toothed belt conducted in the vertical direction laterally of the housing, where the teeth interval has been dimensioned so as to match the plate dimensions in the direction of movement of the belt, the belt then entraining with its teeth a plate which has penetrated between the teeth. The retention of the plate on the belt during the transfer can be ensured by means of one or more magnets mounted on the side of the belt, or optionally a cover can be mounted on the side of the belt, so that the plates pass in the narrow interstice between the belt and the cover, from where they cannot drop from the belt.

The movement of the belt or chain acting as a conveyor is preferably stepped with a step length appropriately slightly longer than the image plate dimension in the direction of movement. The conveyor belt or similar will thus grip the closest plate of the plate pack in the housing and remove it from the pack before stopping at the end of the step, and the next plate in the pack is subsequently allowed to press against the conveyor. The conveyor transfers the plate in one or more steps into a position where the reader slide can grip the plate and transfer it to the reading step. The slide may be adapted to make reciprocating movements so as to, after the plate has been read and perhaps subsequently cleared, return the plate onto the conveyor, the conveyor being immobilised over the entire duration of these operations. With its following step, the conveyor removes the plate that has been read from the process, e.g. by simply letting it drop from the conveyor, while simultaneously bringing the following plate to the location of the reader slide.

As mentioned above, the invention also comprises an apparatus for feeding image plates used in intraoral X-ray photography into a reading device for the reading of the images taken on the plate, as defined in claim 1.

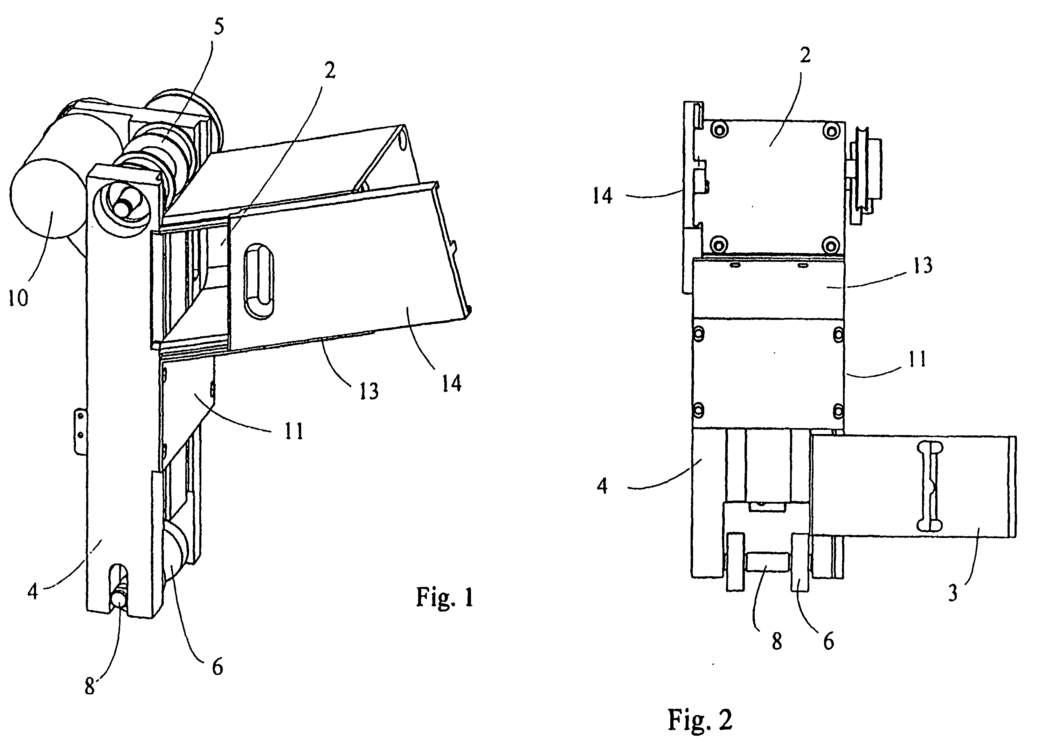

The invention is described in greater detail below by means of an example and with reference to the accompanying drawings, in which
- figure 1: shows an apparatus of the invention for receiving image plates excited in dental X-ray photography and for transferring them within reach of a slide included in a reading device, without conveyor belts integrated in the apparatus,
- figure 2: shows a front view of the apparatus of figure I and said reader slide,
- figure 3: is a view corresponding to figure 2, showing the apparatus including conveyor belts, with the plate housing and the cover parts removed, and
- figure 4: is a cross-section of the entire apparatus along IV-IV of figure 3.

The apparatus illustrated in the drawings is intended to transfer one by one digital image plates 1 excited in intraoral dental X-ray photography for the images to be read by a laser beam. An image plate 1 consists of a thin, rigid or semi-rigid plate, which is preferably mainly made of plastic and which can be equipped with a magnetic metal part in order to utilise magnetism in the shifting of the plate. The surface of the image plate 1 comprises a layer of phosphorus or phosphoric material excited by X-ray photography. In the photographing step, one or more image plates are inserted into the patient's mouth in a plastic bag, which gives protection against saliva and daylight, and in which the X-radiation excites the phosphorus layer on the plate. After the photographing step, the plates 1 are removed from the protective bags and are pushed into the plate housing 2 integrated in the apparatus described. After this, the handling of the plates 1 proceeds automatically so that the apparatus transfers plates one by one from the housing 2 onto the path of the reader slide 3. The slide 3 grips a plate 1 and makes a reciprocating movement, during which the plate is scanned with a laser beam in order to produce the final X-ray photograph on a film or a display terminal, and a clearing light clears the plate from image information for the subsequent X-ray photographing session. The slide 3 resuming its initial position returns the image plate 1 which has been read and cleared onto the path of the apparatus, which then carries out the removal of the plate.

The apparatus described above, in which the image plates 1 are transferred vertically from the top to the bottom, comprises a frame 4, rolls 5, 6 mounted on bearings in the frame and including axes 7, 8 at the upper and lower ends of the apparatus, endless conveyor belts 9 steered by the rolls, and an engine 10 rotating the rolls 5 at the upper end of the apparatus. The conveyor belts 9 form a vertical transfer path for the image plates 1, the path being defined by the plate housing 2 mentioned above and a cover plate 11 below the housing, the cover plate being spaced from the belts by a distance barely allowing the plates to pass between the plate 11 and the belts. Magnets 12 have been placed on the opposite side of the conveyor belts 9 in order to enhance the pressing of the image plates 1 against the belts, enabling these to grip the plate, and to ensure that the plates are retained on the belts until the slide 3 grips the plates in order to transfer them to the image reading step.

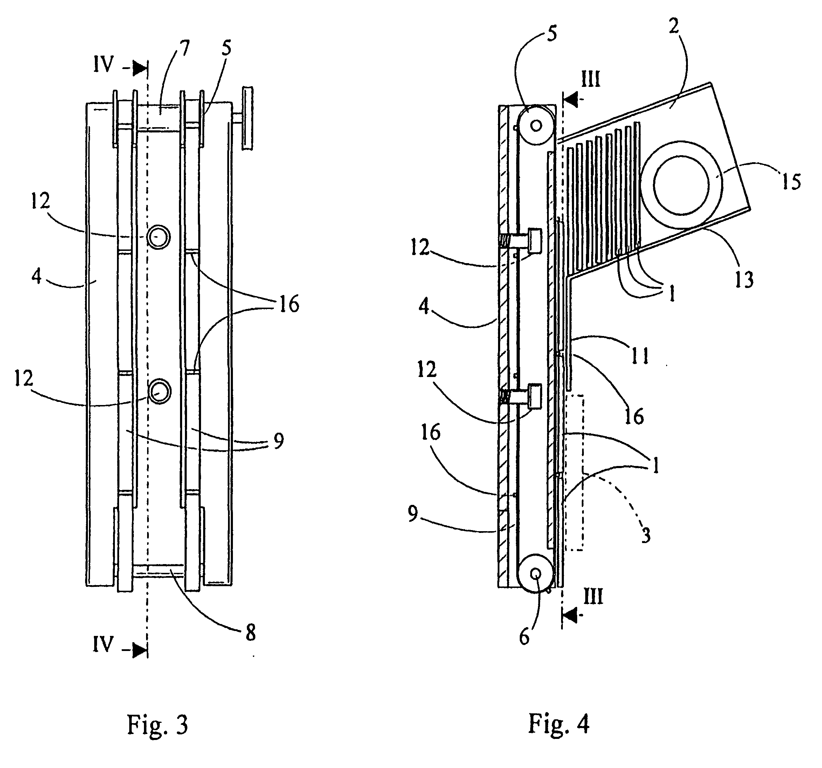

The plate housing 2, where the image plates 1 to be read can be placed in a pack as in figure 4, comprises a bottom 13 inclined towards the conveyor belts 9, a sliding door 14 on the side of the housing, and a freely rolling roll 15, which is placed behind the plate pack in the housing and which pushes the plates 1 towards the belts by force of gravity. The gripping means with which the conveyor belts 9 grip the closest plate 1 of the plate pack in the housing consist of projecting teeth 16, spaced by an interval substantially equal to the width of the rectangular plates placed transversely in the housing. Thus the plate 1 fits into the gap between the teeth of the belt 9, where, pulled by the belts, it moves downwards into a position in the lower part of the apparatus where the reader slide 3 can grip the plate.

The movement of the conveyor belts 9 generated by the engine 10 is stepped in the apparatus, the length of each step equalling a tooth interval, i.e. the distance between the teeth 16. The conveyor belts 9 are immobilised between the steps. The apparatus described here has been dimensioned so that the plate 1 moves from the housing 2 onto the path of the reader slide 3 in two steps. The belts 9 are immobilised over each period that the slide makes its reciprocating movement in order to read and clear the plate. When the slide 3 has returned the plate onto the transfer path formed by the belts 9, the belts take the following step, which brings the subsequent plate to be read to the location of the slide while letting the precedent, already read and cleared plate drop from the belts.

In the apparatus described above, the bottom 13 of the plate housing 2 is inclined, a roll 15 pressing the pack of plates has been placed in the housing, and on the side of the conveyor belts 9 opposite to the plate pack a magnet 12 has been placed, which attracts image plates 1 comprising magnetic metal parts. All of these three factors, which have been exemplified in the drawings, contribute to the pressing of the image plates 1 against the conveyor belts 9 and to the engagement of the plates between the teeth, for the plates to be moved forwards on the transfer path formed by the belts. In the practice, all of these factors are not necessarily called for at the same time, so that it may suffice to provide merely the inclined bottom 13 of the housing 2 or the pushing device 15, or even only a pushing device actuated by a separate actuator in a housing having a horizontal bottom, and then the plates do not require any magnetic parts either. Accordingly, with the use of a magnet 12 behind the pack of plates, the pushing device may be superfluous. The second magnet 12 placed lower in the apparatus, as shown in figures 3 and 4, and the cover plate 11 defining the transfer path of the plates may also offer optional solutions to ensure the retention of the plates.

It is obvious to a person skilled in the art that the various embodiments of the invention may vary also in other ways without departing from the scope of the accompanying claims.

## Claims

1. A method of feeding intraoral dental X-ray image plates (1) used in intraoral dental X-ray photography into a reading device (13) the images taken on the plates to be read, **characterized in that** the method comprises the steps of:
- removing the plates (1) from their casings used during the preceeding intraoral dental X-ray photographing step,
- inserting the plates in a housing (2) provided with a door (14) and substantially protected from light, said housing forming an intermediate storage for the plates,
- stacking the plates to form a pack in said housing, the plates being subjected to a force pulling or pressing them towards a conveyor (9) extending to the external the adjacency of the housing, and
- feeding the plates from said housing by means of a conveyor (9) to the reading device, the conveyor being arranged for gripping each time the plate at the nearest end position of the pack so as to forward the plates in sequence along the feeding path.

2. A method as defined in claim 1, **characterised in that** the bottom (13) of the housing (2) is inclined towards the conveyor (9) so that the plates (1) in the pack are attracted towards the conveyor by force of gravity.

3. A method as defined in claim I or 2, **characterised in that** the pack of plates is pushed towards the conveyor (9) by means of a pushing device, such as a rolling roll (15) located behind the pack.

4. A method as defined in any of the preceding claims, **characterised in that** the image plates (1) comprising a magnetic metal part are attracted towards the conveyor (9) by means of a magnet (12).

5. A method as defined in any of the preceding claims, **characterised in that** the conveyor (9) transfers image plates (1) from the housing (2) onto the path of a slide (3) integrated in the reading device, and that the slide grips the plate entering its path each time and brings the plates one by one to the reading step.

6. A method as defined in claim 5, **characterised in that** the conveyor (9) consists of a belt or a chain conducted laterally of the housing (2).

7. A method as defined in claim 6, **characterised in that** the conveyor comprises a vertically moving toothed belt (9), the image plate (1) fitting between the teeth of the belt so that the belt entrains the plate by means of its teeth (16).

8. A method as defined in claim 6 or 7, **characterised in that** the belt or the chain acting as a conveyor (9) moves stepwise.

9. A method as defined in any of claims 5-8, **characterised in that** the slide (3) of the reading device makes a reciprocating movement, returning a plate ( 1 ) whose image has been read onto the conveyor (9), which subsequently removes the plate from the process.

10. A method as defined in claim 9, **characterised in that** the conveyor (9) transfers plates (1) vertically from the top to the bottom and removes read plates by letting them drop from the conveyor.

11. An apparatus for feeding intraoral dental X-ray image plates (1) used in intraoral dental X-ray photography into a reading device for the images taken on the plates to be read, **characterized in that** the apparatus comprises:
a housing (2) which is adapted for receiving said image plates, provided with a door (14) and substantially protected from light, the housing forming an intermediate storage for the image plates received and stacked therein,
a conveyor (9) extending to the external adjacency of the housing, wherein the plates stacked in the housing are subjected to a force pulling or pressing them towards the conveyor, the conveyor being provided with gripping means (16) adapted to engage with the plates one by one, in order to forward them in sequence along the feeding path.

12. An apparatus as defined in claim 11, **characterised in that** the bottom (13) of the housing (2) is inclined towards the conveyor (9) so that the plates (1) arranged as a pack are attracted towards the conveyor by force of gravity.

13. An apparatus as defined in claim 12, **characterised in that** a freely rolling roll (15) has been disposed in the housing (2) in order to push the pack of plates towards the conveyor (9).

14. An apparatus as defined in any of claims 11-13, **characterised in that** it comprises a magnet (12) in order to attract image plates (1) equipped with a magnetic metal part towards the conveyor (9).

15. An apparatus as defined in any of claims 11-14, **characterised in that** the conveyor (9) comprises a preferably endless belt or chain which is conducted laterally of the housing (2) and moves image plates (1) in sequence onto the path of the slide (3) integrated in the reading device, and **in that** the slide has been disposed to grip the plate entering its path each time, bringing the plates one by one to the image reading step.

16. An apparatus as defined in claims 14 and 15, **characterised in that** the conveyor consists of two parallel belts (9), between which at least one magnet (12) is placed to attract the image plates (1) towards the belts.

17. An apparatus as defined in claim 15 or 16, **characterised in that** the conveyor comprises a belt (9) which moves from the top to the bottom, and that the magnet (12) is placed at a location lower than the bottom (13) of the housing (2) so as to keep the image plates (1) in contact with the belt during the transfer of the plates.

18. An apparatus as defined in any of claims 11-17, **characterised in that** the conveyor is a vertically moving toothed belt (9) with a tooth interval equalling the width of an image plate (1) to allow the plate to fit in-between the teeth.

19. An apparatus as defined in any of claims 11-18, **characterised in that** it comprises a plate-like cover (11) covering the conveyor (9), allowing the image plates (I) to pass between the conveyor and the cover within an interstice dimensioned to equal the thickness of the plates.

## Patentansprüche

1. Verfahren zur Eingabe von intraoralen dentalen Röntgenbildplatten (1), die in intraoraler dentaler Röntgenographie verwendet werden, in ein Lesegerät (3) zum Lesen der auf den Platten aufgenommenen Bilder, **dadurch gekennzeichnet, dass** das Verfahren die Schritte umfasst:
- Entfemen der Platten (1 ), die während eines vorangegangenen intraoralen dentalen Röntgenographieschritts verwendet wurden, aus ihren Umhüllungen,
- Einsetzen der Platten in ein mit einer Tür (14) versehenes und im Wesentlichen lichtgeschütztes Gehäuse (2), wobei das Gehäuse ein Zwischenlager für die Platten darstellt,
- Aufeinanderpacken der Platten, um einen Stapel im Gehäuse zu bilden, wobei die Platten einer Kraft unterworfen sind, die sie in Richtung auf eine Fördereinrichtung (9), die außen benachbart zu dem Gehäuse angeordnet ist, zieht oder schiebt, und
- Zuführen der Platten mittels der Fördereinrichtung (9) vom Gehäuse zum Lesegerät, wobei die Fördereinrichtung so ausgebildet ist, dass sie jeweils diejenige Platte erfasst, die sich in der am nächsten gelegenen Endposition des Stapels befindet, um die Platten nacheinander auf dem Zufuhrweg zu befördern.

2. Verfahren nach Anspruch 1, **dadurch gekennzeichnet, dass** der Boden (13) `des Gehäuses (2) in Richtung auf die Fördereinrichtung (9) geneigt ist, so dass die Platten (1) des Stapels durch die Schwerkraft in Richtung auf die Fördereinrichtung gezogen werden.

3. Verfahren nach Anspruch 1 oder 2, **dadurch gekennzeichnet, dass** der Stapel der Platten durch eine Schiebeeinrichtung, wie beispielsweise eine hinter dem Stapel angeordnete Drehrolle (15) in Richtung auf die Fördereinrichtung (9) geschoben wird.

4. Verfahren nach einem der vorhergehenden Ansprüche, **dadurch gekennzeichnet, dass** die ein magnetisches Metallteil aufweisenden Bildplatten (1) durch einen Magneten (12) in Richtung auf die Fördereinrichtung (9) gezogen werden.

5. Verfahren nach einem der vorhergehenden Ansprüche, **dadurch gekennzeichnet, dass** die Fördereinrichtung (9) die Bildplatten (1) vom Gehäuse (2) auf die Bahn eines Schiebers (3) befördert, der in das Lesegerät integriert ist, und dass der Schieber jeweils die in seine Bahn eintretende Platte ergreift und die Platten Stück für Stück zum Leseschritt bringt.

6. Verfahren nach Anspruch 5, **dadurch gekennzeichnet, dass** die Fördereinrichtung (9) aus einem Riemen oder einer Kette besteht, der bzw. die seitlich am Gehäuse (2) entlang geführt ist.

7. Verfahren nach Anspruch 6, **dadurch gekennzeichnet, dass** die Fördereinrichtung einen sich senkrecht bewegenden Zahnriemen (9) besitzt, wobei die Bildplatte (1) zwischen die Zähne des Riemens passt, so dass der Riemen die Platte mittels seiner Zähne (16) mitnimmt.

8. Verfahren nach Anspruch 6 oder 7, **dadurch gekennzeichnet, dass** der Riemen oder die Kette, der bzw. die als Fördereinrichtung (9) arbeitet, sich schrittweise bewegt.

9. Verfahren nach einem der Ansprüche 5 bis 8, **dadurch gekennzeichnet, dass** der Schieber (3) des Lesegeräts eine Hin- und Herbewegung ausführt, wobei eine Platte (1) deren Bild gelesen wurde, auf die Fördereinrichtung (9) zurück befördert wird, die die Platte anschließend aus dem Arbeitsprozess entfernt.

10. Verfahren nach Anspruch 9, **dadurch gekennzeichnet, dass** die Fördereinrichtung (9) Platten (1) senkrecht von oben nach unten befördert und gelesene Platten entfernt werden, indem die Fördereinrichtung sie herunterfallen lässt.

11. Vorrichtung zur Eingabe von intraoralen dentalen Röntgenbildplatten (1), die in intraoraler dentaler Röntgenographie verwendet werden, in ein Lesegerät zum Lesen der auf den Platten aufgenommenen Bilder, **dadurch gekennzeichnet, dass** die Vorrichtung umfasst:
- ein mit einer Tür (14) versehenes und im Wesentlichen lichtgeschütztes Gehäuse (2), das so ausgebildet ist, dass es die Bildplatten aufnehmen kann, wobei das Gehäuse ein Zwischenlager für die darin aufgenommenen und gestapelten Platten darstellt,
- eine Fördereinrichtung (9), die sich außen benachbart zu dem Gehäuse erstreckt, wobei die in dem Gehäuse aufgestapelten Platten einer Kraft unterworfen sind, die sie in Richtung auf die Fördereinrichtung zieht oder schiebt, und wobei die Fördereinrichtung mit Greifelementen (16) versehen ist, die so ausgebildet sind, dass sie die Platten Stück für Stück greifen, um sie nacheinander auf dem Zufuhrweg zu befördern.

12. Vorrichtung nach Anspruch 11, **dadurch gekennzeichnet, dass** der Boden (13) des Gehäuses (2) in Richtung auf die Fördereinrichtung (9) geneigt ist, so dass die als Stapel angeordneten Platten (1) durch die Schwerkraft in Richtung auf die Fördereinrichtung gezogen werden.

13. Vorrichtung nach Anspruch 12, **dadurch gekennzeichnet, dass** eine frei ' drehbare Rolle (15) im Gehäuse (2) angeordnet ist, um den Stapel Platten in Richtung auf die Fördereinrichtung (9) zu schieben.

14. Vorrichtung nach einem der Ansprüche 11 bis 13, **dadurch gekennzeichnet, dass** sie einen Magneten (12) aufweist, um mit einem magnetischen Metallteil ausgestattete Bildplatten (1) in Richtung auf die Fördereinrichtung zu ziehen.

15. Vorrichtung nach einem der Ansprüche 11 bis 14, **dadurch gekennzeichnet, dass** die Fördereinrichtung (9) vorzugsweise einen endlosen Riemen oder eine Kette umfasst, der bzw. die seitlich am Gehäuse (2) geführt ist und Bildplatten (1) nacheinander auf die Bahn des in das Lesegerät integrierten Schiebers (3) befördert, und dass der Schieber so angeordnet ist, dass er jeweils die in seine Bahn kommende Platte greift, wobei die Platten Stück für Stück zum Bildleseschritt gebracht werden.

16. Vorrichtung nach den Ansprüchen 14 und 15, **dadurch gekennzeichnet, dass** die Fördereinrichtung aus zwei parallelen Riemen (9) besteht, zwischen denen wenigstens ein Magnet (12) angeordnet ist, um die Bildplatten (1 ) in Richtung auf die Riemen zu ziehen.

17. Vorrichtung nach den Ansprüchen 15 oder 16, **dadurch gekennzeichnet, dass** die Fördereinrichtung einen Riemen (9) aufweist, der sich von oben nach unten bewegt, und dass der Magnet (12) an einer Stelle platziert ist, die tiefer als der Boden (13) des Gehäuses (2) liegt, um die Bildplatten (1) während des Transports der Platten in Kontakt mit dem Riemen zu halten.

18. Vorrichtung nach einem der Ansprüche 11 bis 17, **dadurch gekennzeichnet, dass** die Fördereinrichtung ein sich senkrecht bewegender Zahnriemen (9) mit einem Zahnabstand, der der Breite einer Bildplatte (1) entspricht, ist, damit die Platte zwischen die Zähne passt.

19. Vorrichtung nach einem der Ansprüche 11 bis 18, **dadurch gekennzeichnet, dass** sie eine plattenartige Abdeckung (11) zum Abdecken der Fördereinrichtung (9) aufweist, wobei es den Bildplatten (1) durch einen Zwischenraum, der in seinen Abmessungen der Stärke der Platten entspricht, möglich ist, sich zwischen der Fördereinrichtung und der Abdeckung hindurch zu bewegen.

## Revendications

1. Procédé d'alimentation de plaques de radiographie dentaire intraorale utilisées dans la radiographie dentaire intraorale dans un dispositif de lecture qui est agencé pour les images prises sur les plaques à lire, **caractérisé en ce que** le procédé comprend les étapes consistant à :
retirer les plaques (1) de leurs boîtiers utilisés au cours de l'étape de radiographie dentaire intraorale précédente,
insérer les plaques dans un logement (2) pourvu d'une porte (14) et sensiblement protégé de la lumière, ledit logement formant un stockage intermédiaire pour les plaques,
empiler les plaques pour former une pile dans ledit logement, les plaques étant soumises à une force les tirant ou les poussant vers un transporteur (9) s'étendant dans la contiguïté du logement, et
amener les plaques depuis ledit logement à l'aide d'un transporteur (9) vers le dispositif de lecture, le transporteur étant agencé pour saisir à chaque fois la plaque située à la position d'extrémité la plus proche de la pile afin d'acheminer les plaques en séquence le long du chemin d'alimentation.

2. Procédé selon la revendication 1, **caractérisé en ce que** la partie inférieure (13) du logement (2) est inclinée vers le transporteur (9) de sorte que les plaques (1) dans la pile sont attirées vers le transporteur par la force de gravité.

3. Procédé selon la revendication 1 ou 2, **caractérisé en ce que** la pile de plaques est poussée vers le transporteur (9) au moyen d'un dispositif de poussée, tel qu'un cylindre de laminage (15) placé derrière la pile.

4. Procédé selon l'une quelconque des revendications précédentes, **caractérisé en ce que** les plaques d'image (1) comprenant une pièce métallique magnétique sont attirées vers le transporteur (9) au moyen d'un aimant (12).

5. Procédé selon l'une quelconque des revendications précédentes, **caractérisé en ce que** le transporteur (9) transfère les plaques d'image (1) depuis le logement (2) sur le chemin d'une glissière (3) intégrée dans le dispositif de lecture et **en ce que** la glissière saisit à chaque fois la plaque pénétrant sur son chemin et apporte les plaques une par une vers l'étape de lecture.

6. Procédé selon la revendication 5, **caractérisé en ce que** le transporteur (9) est constitué d'une courroie ou d'une chaîne dirigée dans le sens latéral du logement (2).

7. Procédé selon la revendication 6, **caractérisé en ce que** le transporteur comprend une courroie dentée se déplaçant verticalement (9), la plaque d'image (1) s'ajustant entre les dents de la courroie de sorte que la courroie entraîne la plaque à l'aide de ses dents (16).

8. Procédé selon la revendication 6 ou 7, **caractérisé en ce que** la courroie ou la chaîne agissant comme un transporteur (9) se déplace par étapes.

9. Procédé selon l'une quelconque des revendications 5 à 8, **caractérisé en ce que** la glissière (3) du dispositif de lecture effectue un mouvement de va et vient, retournant une plaque (1) dont l'image a été lue sur le transporteur (9), ce qui retire ensuite la plaque du procédé.

10. Procédé selon la revendication 9, **caractérisé en ce que** le transporteur (9) transfère les plaques (1) verticalement de haut en bas et retire les plaques lues en les laissant tomber du transporteur.

11. Dispositif d'alimentation de plaques de radiographie dentaire intraorale (1) utilisé dans la radiographie dentaire intraorale dans un dispositif de lecture pour les images prises sur les plaques à lire, **caractérisé en ce que** le dispositif comprend :
un logement (2) qui est agencé pour recevoir lesdites plaques d'image, pourvu d'une porte (14) et sensiblement protégé de la lumière, le logement formant un stockage intermédiaire pour les plaques d'image reçues et empilées dans celui-ci.
un transporteur (9) s'étendant dans la contiguïté externe du logement, dans lequel les plaques empilées dans le logement sont soumises à une force les tirant ou les poussant vers le transporteur, le transporteur étant pourvu de moyens de saisie (16), adaptés pour se mettre en prise avec les plaques une à une, afin de les acheminer en séquence le long du chemin d'alimentation.

12. Dispositif selon la revendication 11, **caractérisé en ce que** la partie inférieure (13) du logement (2) est inclinée vers le transporteur (9) de sorte que les plaques (1) disposées en pile sont attirées vers le transporteur par la force de gravité.

13. Dispositif selon la revendication 12, **caractérisé en ce qu'**un cylindre de laminage libre (15) a été disposé dans le logement (2) afin de pousser la pile de plaques vers le transporteur (9).

14. Dispositif selon l'une quelconque des revendications 11 à 13, **caractérisé en ce qu'**il comprend un aimant (12) afin d'attirer les plaques d'image (1) équipées d'une pièce métallique magnétique vers le transporteur (9).

15. Dispositif selon l'une quelconque des revendications 11 à 14, **caractérisé en ce que** le transporteur (9) comprend une courroie ou une chaîne de préférence sans fin qui est conduite dans le sens latéral du logement (2) et déplace les plaques d'image (1) en séquence sur le chemin de la glissière (3) intégrée dans le dispositif de lecture et **en ce que** la glissière a été disposée pour saisir à chaque fois la plaque pénétrant sur son chemin, apportant les plaques une par une vers l'étape de lecture de l'image.

16. Dispositif selon les revendications 14 et 15, **caractérisé en ce que** le transporteur est constitué de deux courroies parallèles (9), entre lesquelles au moins un aimant (12) est placé pour attirer les plaques d'image (1) vers les courroies.

17. Dispositif selon la revendication 15 ou 16, **caractérisé en ce que** le transporteur comprend une courroie (9) qui se déplace de haut en bas et **en ce que** l'aimant (12) est placé sur un emplacement plus bas que la partie inférieure (13) du logement (2) afin de maintenir les plaques d'image (1) en contact avec la courroie pendant le transfert des plaques.

18. Dispositif selon l'une quelconque des revendications 11 à 17, **caractérisé en ce que** le transporteur est une courroie dentée se déplaçant verticalement (9) avec un intervalle de dent égal à la largeur d'une plaque d'image (1) pour permettre à la plaque d'être ajustée entre les dents.

19. Dispositif selon l'une quelconque des revendications 11 à 18, **caractérisé en ce qu'**il comprend un capot en forme de plaque (11) recouvrant le transporteur (9), permettant aux plaques d'image (1) de passer entre le transporteur et le capot dans un interstice présentant une dimension égale à l'épaisseur des plaques.
